(19) Europäisches Patentamt
European Patent Office
Office européen des brevets

(11) EP 1 247 815 A2

(12) **EUROPEAN PATENT APPLICATION**

(43) Date of publication:
**09.10.2002 Bulletin 2002/41**

(51) Int Cl.[7]: **C07H 21/00**, C12Q 1/68

(21) Application number: **02388025.5**

(22) Date of filing: **25.03.2002**

(84) Designated Contracting States:
**AT BE CH CY DE DK ES FI FR GB GR IE IT LI LU MC NL PT SE TR**
Designated Extension States:
**AL LT LV MK RO SI**

(30) Priority: **25.03.2001 US 278598 P**

(71) Applicant: **Exiqon A/S**
**2950 Vedbaek (DK)**

(72) Inventors:
- **Jakobsen, Mogens Havsteen**
  **2720 Vanlose (DK)**
- **Kongsbak, Lars**
  **2840 Holte (DK)**
- **Pfundheller, Henrik**
  **2970 Horsholm (DK)**

(54) **Modified oligonucleotides and uses thereof**

(57) Chimeric oligonuclcotides are provided that contain non-modified DNA or RNA residues and modified nucleic acid residues. A modified nucleic acid residue is placed in the -1 position of the 3' and/or 5' end of the oligonucleotide. The oligonucleotides can exhibit significantly enhanced hybridization properties and improved capabilities as primers in nucleic acid extension and amplification reactions.

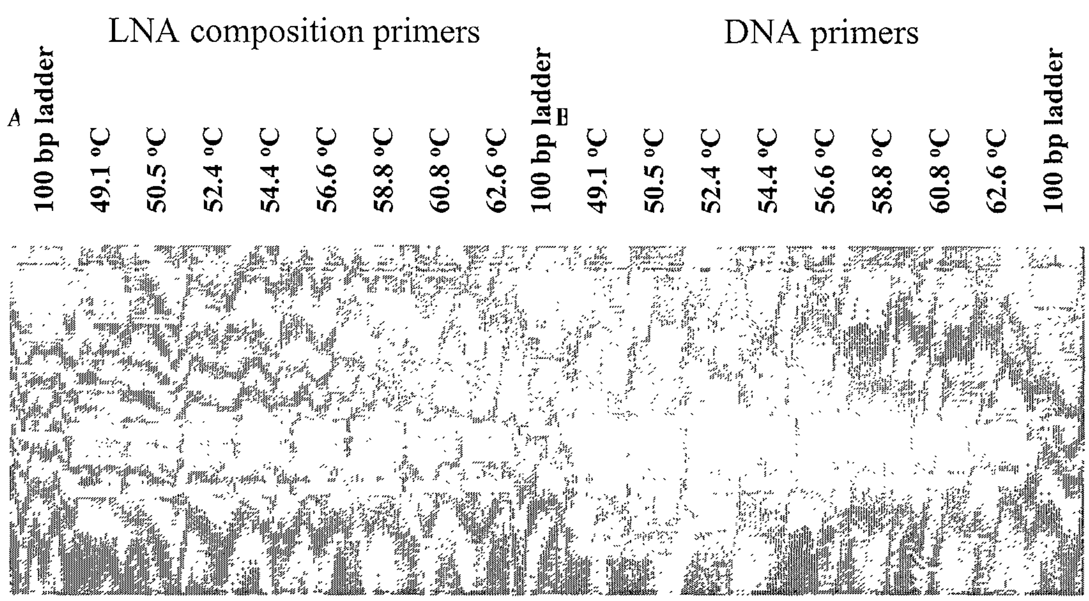


FIG. 1

EP 1 247 815 A2

## Description

FIELD OF THE INVENTION

The invention provides new oligonucleotides that can exhibit significant hybridization properties, including significant discrimination between fully matched target oligonucleotides and oligonucleotides containing one or more mismatches. Also provided are new oligonucleotides showing improved abilities as primers in nucleic acid extension and amplification reactions. Oligonucleotides of the invention may contain a major portion of non-modified or preferably "natural" DNA or RNA residues, and a minor portion comprising one or more other modified nucleic acid-based residues, particularly multicyclic structures such as locked nucleic acids (LNA).

BACKGROUND OF THE INVENTION

In molecular biology, oligonucleotides are routinely used for a variety of purposes such as for example (i) as hybridisation probes in the capture, identification and quantification of target nucleic acids (ii) as affinity probes in the purification of target nucleic acids (iii) as primers in sequencing reactions and target amplification processes such as the polymerase chain reaction (PCR) (iv) to clone and mutate nucleic acids and (vi) as building blocks in the assembly of macromolecular structures.

In therapeutic applications oligonucleotides have been used successfully to block translation in vivo of specific mRNAs thereby preventing the synthesis of proteins, which are undesired or harmful to the cell/organism. This concept of oligonucleotide mediated blocking of translation is known as the "antisense" approach. Mechanistically, the hybridising oligonucleotide is thought to elicit its effect by either creating a physical block to the translation process or by recruiting cellular enzymes that specifically degrades the mRNA part of the duplex (RNase H).

To be useful in the extensive range of different applications outlined above, oligonucleotides have to satisfy a large number of different requirements. In antisense therapeutics, for instance, a useful oligonucleotide must be able to penetrate the cell membrane, have good resistance to extra- and intracellular nucleases and preferably have the ability to recruit endogenous enzymes like RNaseH. In DNA-based diagnostics and molecular biology other properties are important such as, *e.g.,* the ability of oligonucleotides to act as efficient substrates for a wide range of different enzymes evolved to act on natural nucleic acids, such as *e.g.* polymerases, kinases, ligases and phosphatases.

The fundamental property of oligonucleotides, however, which underlies all uses is their ability to recognise and hybridise sequence specifically to complementary single stranded nucleic acids employing either Watson-Crick hydrogen bonding (A-T and G-C) or other hydrogen bonding schemes such as the Hoogsteen mode.

Two important hybridization characteristics of a given oligonucleotide are affinity and specificity. Affinity is a measure of the binding strength of the oligonucleotide to its complementary target sequence (expressed as the thermostability ($T_m$) of the duplex). Specificity is a measure of the ability of the oligonucleotide to discriminate between a fully complementary and a mismatched target sequence. In other words, specificity is a measure of the loss of affinity associated with mismatched nucleobase pairs in the target.

In general, an increase in the affinity of an oligonucleotide occurs at the expense of specificity and vice-versa. This can pose problems with use of oligonucleotides. For instance, in diagnostic procedures, the oligonucleotide needs to have both high affinity to secure adequate sensitivity of the test and high specificity to avoid false positive results. Similarly, an oligonucleotide used as antisense probes needs to have both high affinity for its target mRNA to efficiently impair its translation and high specificity to avoid the unintentional blocking of the expression of other proteins. With enzymatic reactions, like, *e.g.,* PCR amplification, the affinity of the oligonucleotide primer must be sufficiently high for the primer/target duplex to be stable in the temperature range where the enzymes exhibits activity, and specificity also needs to be sufficiently high to ensure that only the correct target sequence is amplified.

It thus would be desirable to have new oligonucleotides. It would be particularly desirable to have new oligonucleotides that exhibit enhanced specificity and enhanced abilities as primers over prior systems.

SUMMARY OF THE INVENTION

We have now discovered new chimeric oligonucleotides that can provide significantly enhanced hybridization and priming properties.

In particular, we have found that oligonucleotides of the invention can provide surprisingly enhanced results upon use as PCR primers. See, for instance, the results set forth in the Example 1, which follow. Oligonucleotides of the invention can also provide exceptional discrimination between fully complementary target sequences and sequences having one or more mismatches. See the results of Example 2, which follows.

According to the present invention an oligonucleotide is provided which comprises non-modified nucleic acid residues and modified nucleic acid residues,

wherein the -1 residue of the oligonucleotides 3' and/or 5' ends is a modified nucleic acid. Preferred oligonucleotides will contain a non-modified DNA or RNA residues at 3' and/or 5' ends and a modified DNA or RNA residue at one position in from (generally referred to herein as the -1 position) either or both the 3' and 5' terminal non-modified nucleic acid residues.

In some embodiments of the present invention it may be of advantage to use further modified nucleic acids. In particular oligonucleotides of the invention may contain a major portion (particularly greater than 50 percent of total oligonucleotide residues) of non-modified DNA or RNA residues and a minor portion of modified nucleic acid residues.

A variety of modified nucleic acids may be employed in oligonucleotides of the invention. Generally preferred modified nucleic acids have the ability of increasing the affinity of the oligonucleotide to a hybridization partner. Generally, increased affinity can be determined by increased $T_m$. Specifically preferred modified nucleic acids for use as units of oligonucleotides of the invention include locked nucleic acids (which include bicyclic and tricyclic DNA or RNA having a 2'-4' or 2'-3' sugar linkages); 2'-deoxy-2'-fluoro ribonucleotides; 2'-*O*-methyl ribonucleotides; 2'-*O*-methoxyethyl ribonucleotides; peptide nucleic acids; 5-propynyl pyrimidine ribonucleotides; 7-deazapurine ribonucleotides; 2,6-diaminopurine ribonucleotides; and 2-thio-pyrimidine ribonucleotides.

The invention also includes oligonucleotides designed for a particular application. Particularly, the design of the oligonucleotide may be adapted for use as a capture probe in a SNP assay, as a Taqman probe, Molecular Beacon, a primer in extension or amplification reaction, and the like. In some aspects of the present invention the oligonucleotide may serve as a capture probe as well as a primer. Oligonucleotides of the invention for use in various nucleic acid manipulation reactions, particularly nucleic acid amplification reactions, polymerase chain reactions (PCR), including singleplex and multiplex PCR, are also provided.

Oligonucleotides of the invention for SNP detection is in general optimized for discrimination between fully complementary target sequences and sequences having one or more mismatches. In a preferred embodiment such oligonucleotides comprise a modified nucleic acid residue at a position opposite to the mismatch position of the target sequence. In a still more preferred embodiment, one or both nucleic acid residues flanking the modified nucleic acid positioned opposite the mismatch position of the target sequence are modified nucleic acid residues. The modified nucleic acid positioned opposite the mismatch position of the target sequence may be comprised in a consecutive stretch of 3, 4, 5, or 6 modified nucleic acids. Generally, it is not preferred with extended stretches of modified nucleic acid moieties because the high affinity may result in unspecific binding. That is, preferably one or more non-modified DNA or RNA will be present after a consecutive stretch of about 3, 4, 5 or 6 modified nucleic acids.

Assay systems are also provided which generally comprise an assay substrate platform packaged with or otherwise containing one or more oligonucleotides of the invention. The one or more oligonucleotides may be immobilized, e.g. by a covalent linkages, to the substrate surface. The assay may be used to conduct e.g. a diagnostic test, e.g. genotyping or detection of a disease marker from a fluid sample (e.g. patient's blood, urine or the like).

Other aspects of the invention are discussed *infra*.

## BRIEF DESCRIPTION OF THE DRAWINGS

FIG. 1 shows results of Example 1 which follows, particularly the products of multiplex PCR reactions run with annealing temperatures from 49.1°C to 62.6°C and quantified by performing line scans along the middle of separate lanes. The left panel of FIG. 1 shows PCR products generated using LNA modified primers (one nucleotide upstream (-1 position) to the 3' end); the right panel of FIG. 1 shows results generated using DNA primers containing no modified nucleic acid residues.

FIG. 2 (which includes FIGS. 2A and 2B) shows results of Example 2, which follows, particularly capture probe discrimination between wt and mt target DNA's. The discrimination ratio was calculated by dividing the capture probe hybridization signal from the matching target with the signal from the mismatching target e.g. $SIGNAL_{wt\ capture\ probe\ +\ wt\ target}$/ $SIGNAL_{wt\ capture\ probe\ +\ mt\ target}$. In FIG. 2, L is LNA (i.e. a modified unit) and d is DNA (i.e. non-modified mer). FIG. 2A shows capture probe discrimination between wt and mt SNP's at HNF1-159. FIG. 2B shows capture probe discrimination between wt and mt at three different SNP's.

## DETAILED DESCRIPTION OF THE INVENTION

As discussed above, new chimeric oligonucleotides are provided that contain a mixture of non-modified nucleic acids and modified (non-natural) nucleic acids.

Oligonucleotides of the invention preferably contain at least 50 percent or more, more preferably 55, 60, 65, or 70 percent or more of non-modified DNA or RNA residues, based on total residues of the oligo. A non-modified

nucleic acid as referred to herein means that the nucleic acid upon incorporation into a 10-mer oligomer will not increase the $T_m$ of the oligomer in excess of 1°C or 2°C. More preferably, the non-modified nucleic acid residue is a substantially or completely "natural" nucleic acid, i.e. containing a non-modified base of uracil, cytosine, thymine, adenine or guanine and a non-modified pentose sugar unit of β-D-ribose (in the case of RNA) or β-D-2-deoxyribose (in the case of DNA).

Oligonucleotides of the invention suitably may contain only a single modified nucleic acid residue, but in some applications, it is preferred that the oligonucleotide will contain 2, 3, 4 or 5 or more modified nucleic acid residues. Typically preferred is where an oligonucleotide contains from about 5 to about 40 or 45 percent modified nucleic acid residues, based on total residues of the oligo, more preferably where the oligonucleotide contains from about 5 or 10 percent to about 20, 25, 30 or 35 percent modified nucleic acid residues, based on total residues of the oligo.

As discussed above, particularly preferred oligonucleotides will contain a non-modified DNA or RNA residues at 3' and/or 5' ends and a modified nucleic acid residue at one position upstream from (generally referred to herein as the -1 position) either or both the 3' and 5' terminal non-modified nucleic acid residues.

Preferred modified nucleic acids have the ability of increasing the affinity of the oligonucleotide to a hybridization partner. Generally, increased affinity can be determined by increased $T_m$. Preferably, a modified nucleic acid will increase the $T_m$ of 15-mer or 20-mer oligo by at least about 1°C or 2°C, more preferably at least about 3, 4 or 5°C.

In one aspect of the invention, the oligonucleotides include those of the following formula I:

$$5'\text{-}X^1\text{-}X^2\text{-}(X^3)_n\text{-}X^4\text{-}X^5\text{-}3' \qquad \text{I}$$

wherein each of $X^1$, $X^2$, $X^3$, $X^4$ and $X^5$ are linked nucleic acid residues; at least one of $X^2$ and $X^4$ is a modified nucleic acid residue; and n is an integer of 0 or greater. Preferably n is from 1 to about 50, more preferably n is 1, 2, 3, 4, 5, 6, 7, 8, 9, 10, 11, 12, 13, 14, 15, 20, 25 or 30. Each $X^3$ may be the same or different.

LNA residues are in general particularly preferred modified nucleic acids for incorporation into an oligonucleotide of the invention. LNAs are described in WO 99/14226, which is incorporated herein by reference. Additionally, the nucleic acids may be modified at either the 3' and/or 5' end by any type of modification known in the art. For example, either or both ends may be capped with a protecting group, attached to a flexible linking group, attached to a reactive group to aid in attachment to the substrate surface, etc.

As disclosed in WO 99/14226, LNA are a novel class of nucleic acid analogues that form DNA- or RNA-heteroduplexes with exceptionally high thermal stability. LNA monomers include bicyclic compounds as shown immediately below:

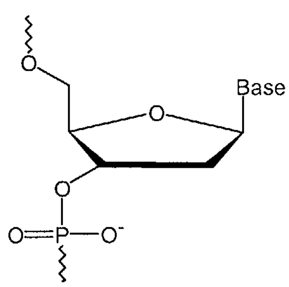

DNA

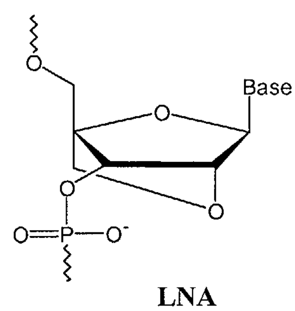

LNA

The LNA moiety depicted above is often referred to as oxy-LNA due to the presence of an oxygen at the 2'-position. Oxy-LNA is especially preferred as modified nucleic acid. References herein to Locked Nucleic Acid, LNA or similar term is inclusive of all such compounds as disclosed in WO 99/14226.

Preferred LNA monomers and oligomers can share chemical properties of DNA and RNA; they are water soluble, can be separated by agarose gel electrophoresis, can be ethanol precipitated, etc.

Introduction of LNA monomers into either DNA, RNA or pure LNA oligonucleotides results in extremely high thermal stability of duplexes with complimentary DNA or RNA, while at the same time obeying the Watson-Crick base pairing rules. In general, the thermal stability of heteroduplexes is increased 3-8°C per LNA monomer in the duplex. Oligonucleotides containing LNA can be designed to be substrates for polymerases (e.g. *Taq* polymerase), and PCR based on LNA primers is more discriminatory towards single base mutations in the template DNA compared to normal DNA-primers (i.e. allele specific PCR).

Oligonucleotides containing LNA are readily synthesized by standard phosphoramidite chemistry. The flexibility of the phosphoramidite synthesis approach further facilitates the easy production of LNA oligos carrying all types of standard linkers, fluorophores and reporter groups.

Particularly preferred LNA monomer for incorporation into an oligonucleotide of the invention include those of the following formula I

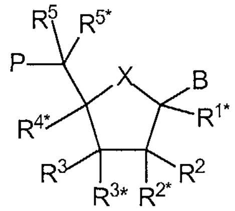

I

wherein X represents oxygen, sulfur, nitrogen, substituted nitrogen, carbon and substituted carbon, and preferably is oxygen; B is a nucleobase; $R^{1*}$, $R^2$, $R^3$, $R^5$ and $R^{5*}$ are hydrogen; P designates the radical position for an internucleoside linkage to a succeeding monomer, or a 5'-terminal group, $R^{3*}$ is an internucleoside linkage to a preceding monomer, or a 3'-terminal group; and $R^{2*}$ and $R^{4*}$ together designate $-O-CH_2-$ where the oxygen is attached in the 2'-position, or a linkage of $-(CH_2)_n-$ where n is 2, 3 or 4, preferably 2, or a linkage of $-S-CH_2-$ or $-NH-CH_2-$.

As used herein, including with respect to formula Ia, the term "nucleobase" covers the naturally occurring nucleobases adenine (A), guanine (G), cytosine (C), thymine (T) and uracil (U) as well as non-naturally occuring nucleobases such as xanthine, diaminopurine, 8-oxo-$N^6$-methyladenine, 7-deazaxanthine, 7-deazaguanine, $N^4$,$N^4$-ethanocytosin, $N^6$,$N^6$-ethano-2,6-diaminopurine, 5-methylcytosine, 5-($C^3$-$C^6$)-alkynylcytosine, 5-fluorouracil, 5-bromouracil, pseudoisocytosine, 2-hydroxy-5-methyl-4-triazolopyridin, isocytosine, isoguanin, inosine and the "non-naturally occurring" nucleobases described in Benner et al., U.S. Pat No. 5,432,272 and Susan M. Freier and Karl-Heinz Altmann, Nucleic Acids Research, 1997, vol. 25, pp 4429-4443. The term "nucleobase" thus includes not only the known purine and pyrimidine heterocycles, but also heterocyclic analogues and tautomers thereof. It should be clear to the person skilled in the art that various nucleobases which previously have been considered "non-naturally occurring" have subsequently been found in nature.

Although LNA monomers having the β-D-ribo configuration are often the most applicable, other configurations also are suitable for purposes of the invention. Of particular use are α-L-ribo, the β-D-xylo and the α-L-xylo configurations (see Beier et al., Science, 1999, 283, 699 and Eschenmoser, Science, 1999, 284, 2118), in particular those having a 2'-4' $-CH_2-S-$, $-CH_2-NH-$, $-CH_2-O-$ or $-CH_2-NMe-$ bridge.

Particularly preferred LNA monomers for use in oligonucleotides of the invention are 2'-deoxyribonucleotides, ribonucleotides, and analogues thereof that are modified at the 2'-position in the ribose, such as 2'-O-methyl, 2'-fluoro, 2'-trifluoromethyl, 2'-O-(2-methoxyethyl), 2'-O-aminopropyl, 2'-O-dimethylamino-oxyethyl, 2'-O-fluoroethyl or 2'-O-propenyl, and analogues wherein the modification involves both the 2'and 3' position, preferably such analogues wherein the modifications links the 2'- and 3'-position in the ribose, such as those described in Nielsen et al., J. Chem. Soc., Perkin Trans. 1, 1997, 3423-33, and in WO 99/14226, and analogues wherein the modification involves both the 2'- and 4'-position, preferably such analogues wherein the modifications links the 2'- and 4'-position in the ribose, such as analogues having a $-CH_2-S-$ or a $-CH_2-NH-$ or a $-CH_2-NMe-$ bridge (see Singh et al. J. Org. Chem. 1998, 6, 6078-9).

In the present context, the term "oligonucleotide" which is the same as "oligomer" which is the same as "oligo" means a successive chain of nucleoside monomers (*i.e.* glycosides of heterocyclic bases) connected via internucleoside linkages. The linkage between two successive monomers in the oligo consist of 2 to 4, preferably 3, groups/atoms selected from $-CH_2-$, -O-, -S-, $-NR^H-$, >C=O, $>C=NR^H$, >C=S, $-Si(R'')_2-$, -SO-, $-S(O)_2-$, $-P(O)_2-$, $-PO(BH_3)-$, -P(O,S)-, $-P(S)_2-$, -PO(R'')-, $-PO(OCH_3)-$, and $-PO(NHR^H)-$, where $R^H$ is selected from hydrogen and $C_{1-4}$-alkyl, and R'' is selected from $C_{1-6}$-alkyl and phenyl. Illustrative examples of such linkages are $-CH_2-CH_2-CH_2-$, $-CH_2-CO-CH_2-$, $-CH_2-CHOH-CH_2-$, $-O-CH_2-O-$, $-O-CH_2-CH_2-$, $-O-CH_2-CH=$ (including $R^5$ when used as a linkage to a succeeding monomer), $-CH_2-CH_2-O-$, $-NR^H-CH_2-CH_2-$, $-CH_2-CH_2-NR^H-$, $-CH_2-NR^H-CH_2-$, $-O-CH_2-CH_2-NR^H-$, $-NR^H-CO-O-$, $-NR^H-CO-NR^H-$, $-NR^H-CS-NR^H-$, $-NR^H-C(=NR^H)-NR^H-$, $-NR^H-CO-CH_2-NR^H-$, -O-CO-O-, $-O-CO-CH_2-O-$, $-O-CH_2-CO-O-$, $-CH_2-CO-NR^H-$, $-O-CO-NR^H-$, $-NR^H-CO-CH_2-$, $-O-CH_2-CO-NR^H-$, $-O-CH_2-CH_2-NR^H-$, -CH=N-O-, $-CH_2-NR^H-O-$, $-CH_2-O-N=$ (including $R^5$ when used as a linkage to a succeeding monomer), $-CH_2-O-NR^H-$, $-CO-NR^H-CH_2-$, $-CH_2-NR^H-O-$, $-CH_2-NR^H-CO-$, $-O-NR^H-CH_2-$, $-O-NR^H-$, $-O-CH_2-S-$, $-S-CH_2-O-$, $-CH_2-CH_2-S-$, $-O-CH_2-CH_2-S-$, $-S-CH_2-CH=$ (including $R^5$ when used as a linkage to a succeeding monomer), $-S-CH_2-CH_2-$, $-S-CH_2-CH_2-O-$, $-S-CH_2-CH_2-S-$, $-CH_2-S-CH_2-$, $-CH_2-SO-CH_2-$, $-CH_2-SO_2-CH_2-$, -O-SO-O-, $-O-S(O)_2-O-$, -O-S

$(O)_2$-$CH_2$-, -O-S$(O)_2$-$NR^H$-, -$NR^H$-S$(O)_2$-$CH_2$-, -O-S$(O)_2$-$CH_2$-, -O-P$(O)_2$-O-, -O-P(O,S)-O-, -O-P$(S)_2$-O-, -S-P$(O)_2$-O-, -S-P(O,S)-O-, -S-P$(S)_2$-O-, -O-P$(O)_2$-S-, -O-P(O,S)-S-, -O-P$(S)_2$-S-, -S-P$(O)_2$-S-, -S-P(O,S)-S-, -S-P$(S)_2$-S-, -O-PO(R")-O-, -O-PO($OCH_3$)-O-, -O-PO($OCH_2CH_3$)-O-, -O-PO($OCH_2CH_2$S-R)-O-, -O-PO($BH_3$)-O-, -O-PO($NHR^N$)-O-, -O-P$(O)_2$-$NR^H$-, -$NR^H$-P$(O)_2$-O-, -O-P(O,$NR^H$)-O-, -$CH_2$-P$(O)_2$-O-, -O-P$(O)_2$-$CH_2$-, and -O-Si$(R")_2$-O-; among which -$CH_2$-CO-$NR^H$-, -$CH_2$-$NR^H$-O-, -S-$CH_2$-O-, -O-P$(O)_2$-O-, -O-P(O,S)-O-, -O-P$(S)_2$-O-, -$NR^H$-P$(O)_2$-O-, -O-P(O,$NR^H$)-O-, -O-PO(R")-O-, -O-PO($CH_3$)-O-, and -O-PO($NHR^N$)-O-, where $R^H$ is selected form hydrogen and $C_{1-4}$-alkyl, and R" is selected from $C_{1-6}$-alkyl and phenyl, are especially preferred. Further illustrative examples are given in Mesmaeker et. al., Current Opinion in Structural Biology 1995, 5, 343-355 and Susan M. Freier and Karl-Heinz Altmann, Nucleic Acids Research, 1997, vol 25, pp 4429-4443. The left-hand side of the internucleoside linkage is bound to the 5-membered ring as substituent P* at the 3'-position, whereas the right-hand side is bound to the 5'-position of a preceding monomer.

The term "succeeding monomer" relates to the neighbouring monomer in the 5'-terminal direction and the "preceding monomer" relates to the neighbouring monomer in the 3'-terminal direction.

Monomers are referred to as being "complementary" if they contain nucleobases that can form hydrogen bonds according to Watson-Crick base-pairing rules (e.g. G with C, A with T or A with U) or other hydrogen bonding motifs such as for example diaminopurine with T, inosine with C, pseudoisocytosine with G, etc.

Preferred oligonucleotides of the invention also may have at least one non-modified nucleic acid located either at or within a distance of no more than three bases from the mismatch position(s) of a complementary oligonucleotide, such as at a distance of two bases from the mismatch position, e.g. at a distance of one base from the mismatch position, e.g. at the mismatch position.

The chimeric oligos of the present invention are highly suitable for a variety of diagnostic purposes such as for the isolation, purification, amplification, detection, identification, quantification, or capture of nucleic acids such as DNA, mRNA or non-protein coding cellular RNAs, such as tRNA, rRNA, snRNA and scRNA, or synthetic nucleic acids, *in vivo* or *in vitro.*

The oligomer can comprise a photochemically active group, a thermochemically active group, a chelating group, a reporter group, or a ligand that facilitates the direct of indirect detection of the oligomer or the immobilisation of the oligomer onto a solid support. Such group are typically attached to the oligo when it is intended as a probe for *in situ* hybridisation, in Southern hydridisation, Dot blot hybridisation, reverse Dot blot hybridisation, or in Northern hybridisation.

When the photochemically active group, the thermochemically active group, the chelating group, the reporter group, or the ligand includes a spacer (K), the spacer may suitably comprise a chemically cleavable group.

In the present context, the term "photochemically active groups" covers compounds which are able to undergo chemical reactions upon irradiation with light. Illustrative examples of functional groups hereof are quinones, especially 6-methyl-1,4-naphtoquinone, anthraquinone, naphtoquinone, and 1,4-dimethyl-anthraquinone, diazirines, aromatic azides, benzophenones, psoralens, diazo compounds, and diazirino compounds.

In the present context "thermochemically reactive group" is defined as a functional group which is able to undergo thermochemically-induced covalent bond formation with other groups. Illustrative examples of functional parts thermochemically reactive groups are carboxylic acids, carboxylic acid esters such as activated esters, carboxylic acid halides such as acid fluorides, acid chlorides, acid bromide, and acid iodides, carboxylic acid azides, carboxylic acid hydrazides, sulfonic acids, sulfonic acid esters, sulfonic acid halides, semicarbazides, thiosemicarbazides, aldehydes, ketones, primary alkohols, secondary alcohols, tertiary alcohols, phenols, alkyl halides, thiols, disulphides, primary amines, secondary amines, tertiary amines, hydrazines, epoxides, maleimides, and boronic acid derivatives.

In the present context, the term "chelating group" means a molecule that contains more than one binding site and frequently binds to another molecule, atom or ion through more than one binding site at the same time. Examples of functional parts of chelating groups are iminodiacetic acid, nitrilotriacetic acid, ethylenediamine tetraacetic acid (EDTA), aminophosphonic acid, etc.

In the present context, the term "reporter group" means a group which is detectable either by itself or as a part of an detection series. Examples of functional parts of reporter groups are biotin, digoxigenin, fluorescent groups (groups which are able to absorb electromagnetic radiation, *e.g.* light or X-rays, of a certain wavelength, and which subsequently reemits the energy absorbed as radiation of longer wavelength; illustrative examples are dansyl (5-dimethylamino)-1-naphthalenesulfonyl), DOXYL (N-oxyl-4,4-dimethyloxazolidine), PROXYL (N-oxyl-2,2,5,5-tetramethylpyrrolidine), TEMPO (N-oxyl-2,2,6,6-tetramethylpiperidine), dinitrophenyl, acridines, coumarins, Cy3 and Cy5 (trademarks for Biological Detection Systems, Inc.), erytrosine, coumaric acid, umbelliferone, texas red, rhodamine, tetramethyl rhodamine, Rox, 7-nitrobenzo-2-oxa-1-diazole (NBD), pyrene, fluorescein, Europium, Ruthenium, Samarium, and other rare earth metals), radioisotopic labels, chemiluminescence labels (labels that are detectable via the emission of light during a chemical reaction), spin labels (a free radical (*e.g.* substituted organic nitroxides) or other paramagnetic probes (*e.g.* $Cu^{2+}$, $Mg^{2+}$) bound to a biological molecule being detectable by the use of electron spin resonance spectroscopy), enzymes (such as peroxidases, alkaline phosphatases, β-galactosidases, and glycose oxidases), antigens, antibodies, haptens (groups which are able to combine with an antibody, but which cannot initiate an immune response by itself, such as peptides and steroid hormones), carrier systems for cell membrane penetration such as: fatty acid residues, steroid moieties (cholesteryl), vitamin A, vitamin D, vitamin E, folic acid peptides for specific receptors, groups for mediating endocytose, epidermal growth factor (EGF), bradykinin, and platelet derived growth factor (PDGF). Especially interesting examples are biotin, fluorescein, Texas Red, rhodamine, dinitrophenyl, digoxigenin, Ruthenium, Europium, Cy5, Cy3, etc.

In the present context "ligand" means something, which binds. Ligands can comprise functional groups such as: aromatic groups (such as benzene, pyridine, naphtalene, anthracene, and phenanthrene), heteroaromatic groups (such as thiophene, furan, tetrahydrofuran, pyridine, dioxane, and pyrimidine), carboxylic acids, carboxylic acid esters, carboxylic acid halides, carboxylic acid azides, carboxylic acid hydrazides, sulfonic acids, sulfonic acid esters, sulfonic acid halides, semicarbazides, thiosemicarbazides, aldehydes, ketones, primary alcohols, secondary alcohols, tertiary alcohols, phenols, alkyl halides, thiols, disulphides, primary amines, secondary amines, tertiary amines, hydrazines, epoxides, maleimides, $C_1$-$C_{20}$ alkyl groups optionally interrupted or terminated with one or more heteroatoms such as oxygen atoms, nitrogen atoms, and/or sulphur atoms, optionally containing aromatic or mono/polyunsaturated hydrocarbons, polyoxyethylene such as polyethylene glycol, oligo/polyamides such as poly-$\alpha$-alanine, polyglycine, polylysine, peptides, oligo/polysaccharides, oligo/polyphosphates, toxins, antibiotics, cell poisons, and steroids, and also "affinity ligands", *i.e.* functional groups or biomolecules that have a specific affinity for sites on particular proteins, antibodies, poly- and oligosaccharides, and other biomolecules.

It should be understood that the above-mentioned specific examples under DNA intercalators, photochemically active groups, thermochemically active groups, chelating groups, reporter groups, and ligands correspond to the "active/functional" part of the groups in question. For the person skilled in the art it is furthermore clear that DNA intercalators, photochemically active groups, thermochemically active groups, chelating groups, reporter groups, and ligands are typically represented in the form M-K- where M is the "active/functional" part of the group in question and where K is a spacer through which the "active/functional" part is attached to the 5- or 6-membered ring. Thus, it should be understood that the group B, in the case where B is selected from DNA intercalators, photochemically active groups, thermochemically active groups, chelating groups, reporter groups, and ligands, has the form M-K-, where M is the "active/functional" part of the DNA intercalator, photochemically active group, thermochemically active group, chelating group, reporter group, and ligand, respectively, and where K is an optional spacer comprising 1-50 atoms, preferably 1-30 atoms, in particular 1-15 atoms, between the 5- or 6-membered ring and the "active/functional" part.

In the present context, the term "spacer" means a thermochemically and photochemically non-active distance-making group and is used to join two or more different moieties of the types defined above. Spacers are selected on the basis of a variety of characteristics including their hydrophobicity , hydrophilicity, molecular flexibility and length (*e. g*. see Hermanson et. al., "Immobilized Affinity Ligand Techniques", Academic Press, San Diego, California (1992), p. 137-ff). Generally, the length of the spacers are less than or about 400 Å, in some applications preferably less than 100 Å. The spacer, thus, comprises a chain of carbon atoms optionally interrupted or terminated with one or more heteroatoms, such as oxygen atoms, nitrogen atoms, and/or sulphur atoms. Thus, the spacer K may comprise one or more amide, ester, amino, ether, and/or thioether functionalities, and optionally aromatic or mono/polyunsaturated hydrocarbons, polyoxyethylene such as polyethylene glycol, oligo/polyamides such as poly-$\alpha$-alanine, polyglycine, polylysine, and peptides in general, oligosaccharides, oligo/polyphosphates. Moreover the spacer may consist of combined units thereof. The length of the spacer may vary, taking into consideration the desired or necessary positioning and spatial orientation of the "active/functional" part of the group in question in relation to the 5- or 6-membered ring. In particularly interesting embodiments, the spacer includes a chemically cleavable group. Examples of such chemically cleavable groups include disulphide groups cleavable under reductive conditions, peptide fragments cleavable by peptidases, etc.

As discussed above, oligonucleotides of the invention may be used in high specificity oligo arrays e.g. wherein a multitude of different oligos are affixed to a solid surface in a predetermined pattern (Nature Genetics, suppl. vol. 21, Jan 1999, 1-60 and WO 96/31557). The usefulness of such an array, which can be used to simultaneously analyse a large number of target nucleic acids, depends to a large extend on the specificity of the individual oligos bound to the surface. The target nucleic acids may carry a detectable label or be detected by incubation with suitable detection probes which may also be an oligonucleotide of the invention.

An additional object of the present invention is to provide oligos with combines an increased ability to discriminate between complementary and mismatched targets with the ability to act as substrates for nucleic acid active enzymes such as for example DNA and RNA polymerases, ligases, phosphatases. Such oligos may be used for instance as primers for sequencing nucleic acids and as primers in any of the several well known amplification reactions, such as the PCR reaction.

In a further aspect, oligonucleotides of the invention may be used to construct new affinity pairs, which exhibit enhanced specificity towards each other. The affinity constants can easily be adjusted over a wide range and a vast number of affinity pairs can be designed and synthesised. One part of the affinity pair can be attached to the molecule

of interest (*e.g.* proteins, amplicons, enzymes, polysaccharides, antibodies, haptens, peptides, etc.) by standard methods, while the other part of the affinity pair can be attached to *e.g.* a solid support such as beads, membranes, microtiter plates, sticks, tubes, etc. The solid support may be chosen from a wide range of polymer materials such as for instance polypropylene, polystyrene, polycarbonate or polyethylene. The affinity pairs may be used in selective isolation, purification, capture and detection of a diversity of the target molecules.

Oligonucleotides of the invention also may be employed as probes in the purification, isolation and detection of for instance pathogenic organisms such as vira, bacteria and fungi etc. Oligonucleotides of the invention also may be used as generic tools for the purification, isolation, amplification and detection of nucleic acids from groups of related species such as for instance rRNA from gram-positive or gram negative bacteria, fungi, mammalian cells etc.

Oligonucleotides of the invention also may be employed as an aptamer in molecular diagnostics, e.g. in RNA mediated catalytic processes, in specific binding of antibiotics, drugs, amino acids, peptides, structural proteins, protein receptors, protein enzymes, saccharides, polysaccharides, biological cofactors, nucleic acids, or triphosphates or in the separation of enantiomers from racemic mixtures by stereospecific binding.

Oligonucleotides of the invention also may be used for labeling of cells, e.g. in methods wherein the label allows the cells to be separated from unlabelled cells.

Oligonucleotides also may be conjugated to a compound selected from proteins, amplicons, enzymes, polysaccharides, antibodies, haptens, and peptides.

Kits are also provided containing one or more oligonucleotides of the invention for the isolation, purification, amplification, detection, identification, quantification, or capture of natural or synthetic nucleic acids. The kit typically will contain a reaction body, e.g. a slide or biochip. One or more oligonucleotides of the invention may be suitably immobilized on such a reaction body.

The invention also provides methods for using kits of the invention for carrying out a variety of bioassays. Any type of assay wherein one component is immobilized may be carried out using the substrate platforms of the invention. Bioassays utilizing an immobilized component are well known in the art. Examples of assays utilizing an immobilized component include for example, immunoassays, analysis of protein-protein interactions, analysis of protein-nucleic acid interactions, analysis of nucleic acid-nucleic acid interactions, receptor binding assays, enzyme assays, phosphorylation assays, diagnostic assays for determination of disease state, genetic profiling for drug compatibility analysis, SNP detection, etc.

Identification of a nucleic acid sequence capable of binding to a biomolecule of interest can be achieved by immobilizing a library of nucleic acids onto the substrate surface so that each unique nucleic acid was located at a defined position to form an array. The array would then be exposed to the biomolecule under conditions which favored binding of the biomolecule to the nucleic acids. Non-specifically binding biomolecules could be washed away using mild to stringent buffer conditions depending on the level of specificity of binding desired. The nucleic acid array would then be analyzed to determine which nucleic acid sequences bound to the biomolecule. Preferably the biomolecules would carry a fluorescent tag for use in detection of the location of the bound nucleic acids.

Assay using an immobilized array of nucleic acid sequences may be used for determining the sequence of an unknown nucleic acid; single nucleotide polymorphism (SNP) analysis; analysis of gene expression patterns from a particular species, tissue, cell type, etc.; gene identification; etc.

Oligonucleotides of the invention may also be used for therapeutic applications, e.g. as an antisense, antigene or ribozyme therapeutic agents. In these therapeutic methods, one or more oligonucleotides of the invention is administered as desired to a patient suffering from or susceptible the targeted disease or disorder, e.g. a viral infection.

The following non-limiting examples are illustrative of the invention. All documents mentioned herein are incorporated herein by reference in their entirety.

<u>Example 1</u>: Improved PCR performance with modified oligonucleotide of the invention.

Oligonucleotide PCR primers were prepared that had less than 50 percent modified nucleic acids, based on total residues of the primer. For singleplex and multiplex PCR reactions, the following primers were used - either all DNA or containing one LNA modification at the 3' end or one nucleotide upstream to the 3' end:

| Amplicon | Forward primer sequence | Reverse primer sequence |
|---|---|---|
| Apo B71 | ctctgcagcttcatcctgaa | agggttgaagccatacacct |
| Apo B591 | taataacatggtgtgtcagc | atgacagttggaagttgaga |
| Apo B2488 | atcaattggttacaggaggct | gtccatttgatacattcggtc |
| Apo | aatgattttcaagttcctgacc | ccaaaagtaggtacttcaatt |

(continued)

| Amplicon | Forward primer sequence | Reverse primer sequence |
|---|---|---|
| B2712 | | gtg |
| Apo B3500 | ttcaggaactattgctagtg | acttcaaggttccagatatc |
| Apo B4154 | gtttccagggactcaaggat | gtgagtcaatcagatgcttg |

The PCR reactions were performed under standard conditions (200 μM dNTP, 3 mM $MgCl_2$, 1.25 U AmpliTaq Gold® polymerase, 300 nM forward and reverse primer, respectively), and the same PCR program was used for singleplex as well as multiplex PCR (*denaturing*: 5 min @ 95°C; *amplification* (35 cycles): denature 1 min @ 94°C, anneal 1 min @ 57°C, extend 1 min @ 72°C; *final extension* 30 min @ 60°C, ∞ @ 4°C). Chromosomal DNA from human was used as template at a concentration of 10pg/μl. The PCR products were subsequently run on a 4% agarose gel in 1xTBE.

In singleplex as well as multiplex PCR reactions the modified primers containing an LNA modification at the very 3' position generally worked less well compared to DNA primers as the amount of PCR product was reduced. In contrast, when the LNA molecule was placed one nucleotide upstream (i.e. -1 position) to the 3' end of the primer, the singleplex PCR reaction gave bands of an intensity comparable to or higher than those obtained using DNA primers. This was completely unexpected and suggests that the position of the LNA modification in the PCR primer is crucial.

The effects of such modified primers were subsequently investigated in multiplex PCR reactions and several reactions were performed at varying annealing temperatures (from 49.1°C to 62.6°C). FIG. 1 shows the specificity gained from using LNA in primers for multiplex PCR amplification. Including LNA in the primers gave the expected number of bands without any unspecific amplification over a broad spectrum of temperatures. In fact, a working window of at least 6°C was obtained. However, in the case where pure DNA primers were used no workable multiplex PCR protocol could be obtained due to unspecific priming or loss of amplicons (FIG. 1, right side).

In FIG. 1, the products of multiplex PCR reactions run with annealing temperatures from 49.1°C to 62.6°C as shown on the image. PCR products using LNA modified primers (one nucleotide upstream to the 3' end) on the left side and PCR products generated using DNA primers the right.

The 3' end generates narrower peaks, representing the distinct bands seen in the gel. It is also evident from FIG. 1 that the number of unspecific bands is extremely low and that annealing temperature has little effect on the outcome of the multiplex PCR reaction. In contrast, when only DNA primers are used, the number of unspecific bands is very high and highly temperature dependent.

These results show that, when using primers modified with LNA one nucleotide upstream to the 3' end, several unexpected advantages follow. A large window of optimal annealing temperatures (52.4° to 58.8°C in the present experiment) was obtained, compared to DNA primers which did not support any workable multiplex protocol. Additionally, the modified oligonucleotide of the invention provided sharper bands with extremely low background.

<u>Example 2</u>. SNP genotyping with capture probes of the invention.

Hybridization experiments were performed with oligonucleotides of the invention and comparative oligonucleotides having all non-modified residues as well as greater than 50 percent modified residues.

The capture probes were designed as 8- to 18-mer oligonucleotides with varying LNA content. The lengths of the oligonucleotides were adjusted according to the LNA content to maintain similar affinities of the capture probes:

| | |
|---|---|
| LNA capture probes (100% LNA) | 8-mers |
| DNA and LNA-containing mixmer capture probes | 10 or 12-mers |
| DNA capture probes (100% DNA) | 18-mers |

The sequence of all capture probes was complementary to the sequence encompassing the SNP, which they were designed to detect. For the mixmer capture probes, the polymorphism was localized within a block of 3 LNA's in the center of the oligonucleotide. Further, the mixmer contained LNA moieties one nucleotide from the 3' and 5' end. The 8- to 18-mer capture probe oligonucleotides were synthesized with a 5' anthraquinone (AQ) modification, followed by a oligo$(dT)_{15}$ DNA linker.

The capture probes were diluted to a 10 μM final concentration, and spotted on Euray microarray slides using the Biochip Arrayer One (Packard Biochip Technologies) with 400 μm between spots. The capture probes were immobilized onto the microarray slide by UV irradiation in a Stratalinker (Stratagene). Non-immobilized capture probe oligonucleotides were removed by washing the slides for 2 hours in milli-Q $H_2O$. After drying the slides, 30 µL of a 0.01 µM target DNA solution in 1x SSCT was pipetted onto the hybridization area of the slide. The target DNA consisted of 5' biotin-labeled 50-mer or 30-mer oligonucleotides, each encompassing 1 to 5 SNPs. In each target oligonucleotide, the nucleotide in the individual SNP corresponded either to the wildtype (wt) or the mutant (mt) genotypes. Only one target oligo was hybridized at a time. A cover slip was laid over the hybridization area and the hybridization was performed for 2 hours at 37°C in a moisture chamber. Following hybridization, the slides were washed in 0.15x SSCT at 37°C for 2 hours. Hybridized target DNAs were detected by incubating slides with 30 µL of a 2.5µg/mL streptavidin-Cy5 conjugate in 1x SSCT for 30 min at 37°C, followed by washing of the slides for 1 hour at 37°C in 1x SSCT. The target DNAs hybridized to spots with the capture probe were visualized by scanning the slides in an ArrayWoRx Scanner (Applied Precision). The level of hybridization at each spot was estimated by quantifying the Cy5 signals using the ArrayVision image analysis software. The capture probe designs were evaluated by their ability to discriminate between their 100 percent matched target and the target with the corresponding mismatching SNP. Capture probes synthesized with 100 percent modified residues or no modified residues were inferior to capture probes containing modified residues in an amount of 50 percent of total residues of the oligonucleotides. This was shown by testing three different designs for both wt and mt polymorphisms at the SNP159 in the human nuclear factor 1 HNF1-159 (FIG. 2A). An LNA content of 50% was superior for the tested 10-mer at this SNP.

To test the optimal concentration of LNA moieties at and surrounding the SNP position, three new oligonucleotides were synthesized with a concentration of 25%, 33%, and 42% respectively. The results are shown in FIG. 2B. The optimal discrimination between the wt and mt for three capture probes appears to be in the range of 25% LNA to 42% LNA.

The invention has been described in detail with reference to preferred embodiments thereof. However, it will be appreciated that those skilled in the art, upon consideration of this disclosure, may make modifications and improvements within the spirit and scope of the invention.

## Claims

1. An oligonucleotide comprising non-modified nucleic acid residues and modified nucleic acid residues, wherein the -1 residue of the oligonucleotides 3' and/or 5' ends is a modified nucleic acid.

2. The oligonucleotide according to claim 1, wherein the -1 residue of the oligonucleotides 3' end is a modified nucleic acid.

3. The oligonucleotide according to claim 1 or 2, wherein the -1 residues of the oligonucleotides 3' and 5' ends are a modified nucleic acid.

4. The oligonucleotide according to any of the claims 1 to 3, comprising further modified nucleic acids

5. The oligonucleotide according to any of the claims 1 to 4, wherein greater than 50 percent of the total residues of the oligonucleotide are non-modified nucleic acids.

6. The oligonucleotide according to claims 1 to 5, comprising from 5 to 45 percent modified nucleic acid residues, based on total residues of the oligonucleotide.

7. The oligonucleotide according to any of the claims 1 to 6 suitable for use as a primer.

8. The oligonucleotide according to claim 7, wherein the primer is adapted for use in an extension reaction involving a nucleic acid active enzyme.

9. The oligonucleotide according to claim 7, wherein the primer is adapted for use in a nucleic acid amplification reaction.

10. The oligonucleotide according to claim 9, wherein the nucleic acid amplification reaction is a multiplex polymerase chain reaction (PCR).

11. The oligonucleotide according to any of the claims 1 to 10, for discriminating between fully complementary target sequences and sequences having one or more mismatches, said oligonucleotide comprising a modified nucleic

acid residue at a position opposite to the mismatch position of the target sequence.

**12.** The oligonucleotide according to claims 11, wherein one or both nucleic acid residues flanking the modified nucleic acid positioned opposite the mismatch position of the target sequence are modified nucleic acid residues.

**13.** The oligonucleotide according to any of the claims 1 to 12, comprising a consecutive stretch of 3, 4, 5, or 6 modified nucleic acid residues.

**14.** The oligonucleotide according to any of the claims 11 to 13, wherein the modified nucleic acid positioned opposite the mismatch position of the target sequence is comprised in the consecutive stretch of modified nucleic acid residues.

**15.** The oligonucleotide of any one of claims 1 through 14, wherein the oligonucleotide contains from about 5 to about 100 total residues.

**16.** The oligonucleotide of any one of claims 1 through 15, wherein the oligonucleotide contains from about 5 to about 50 total residues.

**17.** The oligonucleotide of any one of claims 1 through 16 wherein the oligonucleotide contains from about 5 to about 30 total residues.

**18.** The oligonucleotide of any one of claims 1 through 17 wherein the oligonucleotide contains from about 8 to about 15 total residues.

**19.** The oligonucleotide of any one of claims 1 through 18, wherein a modified nucleic acid residue of the oligonucleotide contains a modification at the 2'-position in the ribose.

**20.** The oligonucleotide of any one of claims 1 through 19, wherein the modified nucleic acid residue is an LNA residue.

**21.** The oligonucleotide of any one of claims 1 through 20, wherein the modified nucleic acid residue is an oxy-LNA residues.

**22.** The oligonucleotide of any one of claims 1 through 19, wherein the modified nucleic acid residue is selected from the group consisting of 2'-deoxy-2'-fluoro ribonucleotides, 2'-*O*-methyl ribonucleotides, 2'-*O*-methoxyethyl ribonucleotides, peptide nucleic acids, 5-propynyl pyrimidine ribonucleotides, 7-deazapurine ribonucleotides, 2,6-diaminopurine ribonucleotides, and 2-thio-pyrimidine ribonucleotides.

**23.** The oligonucleotide of any one of claims 1 through 22, wherein the non-modified residues contain deoxyribonucleotides.

**24.** The oligonucleotide of any one of claims 1 through 23 wherein the oligonucleotide is conjugated to one part of an affinity pair or to a compound selected from proteins, amplicons, enzymes, polysaccharides, antibodies, haptens, and peptides.

**25.** The oligonucleotide of any one of claims 1 through 24, wherein the oligonucleotide contains a fluorophor moiety and a quencher moiety, positioned in such a way that the hybridised state of the oligonucleotide can be distinguished from the unbound state of the oligonucleotide by an increase in the fluorescent signal from the nucleotide.

**26.** The oligonucleotide of claim 25, wherein the oligonucleotide is adapted for use as a Taqman probe or Molecular Beacon.

**27.** Use of an oligonucleotide according to any of the claims 1 to 24 as a capture probe in a SNP assay.

**28.** Use of an oligonucleotide according to any of the claims 1 to 24 as a primer in a nucleic acid extension reaction.

**29.** Use of an oligonucleotide according to any of the claims 1 to 24 as a primer in a polymerase chain reaction (PCR).

**30.** Use according to claim 29, wherein multiple primers are used in multiplex PCR.

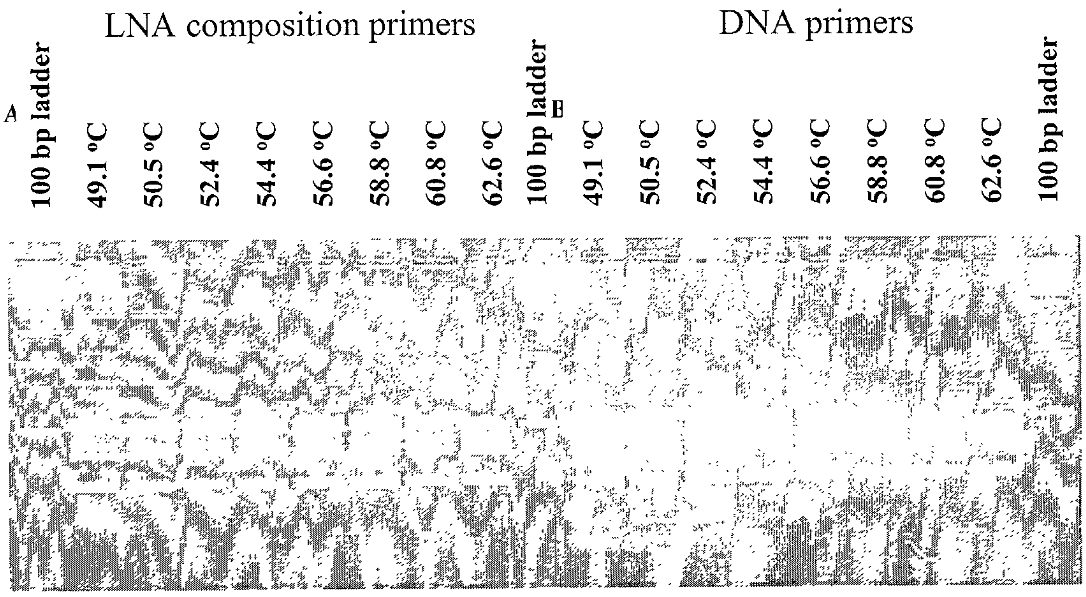


FIG. 1

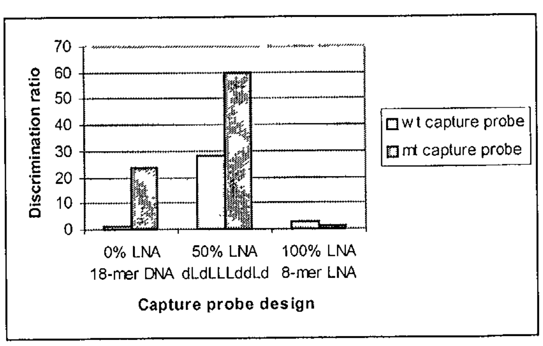


FIG. 2A

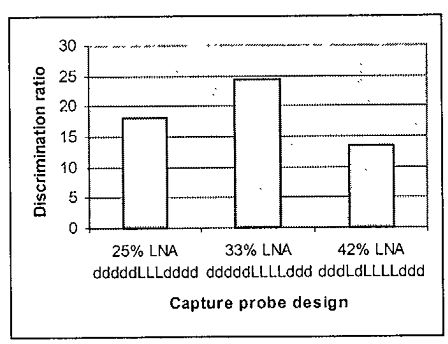


FIG. 2B